# EUROPÄISCHE PATENTSCHRIFT

(11) **EP 4 360 735 B1**
(45) Veröffentlichungstag und Bekanntmachung des Hinweises auf die Patenterteilung: **12.08.2026**
(21) Anmeldenummer: 23206426.1
(22) Anmeldetag: 23.03.2022
(51) Int. Cl.: B01D 47/06, C07C 273/16, B01D 53/00, C05C 9/00

(54) **ANLAGE UND VERFAHREN ZUR HERSTELLUNG VON HARNSTOFFGRANULAT**
PLANT AND METHOD FOR PRODUCING UREA GRANULES
INSTALLATION ET PROCÉDÉ DE FABRICATION DE GRANULES D'URÉE

(30) Priorität: 24.03.2021 DE 102021202869
(43) Veröffentlichungstag der Anmeldung: 01.05.2024
(62) Dokumentnummer(n) der früheren Anmeldung(en) nach Art. 76 EPÜ: 22717181.6 / 4 313 367
(73) Patentinhaber: thyssenkrupp Uhde Fertilizer Technology GmbH, 44141 Dortmund (DE); thyssenkrupp AG, 45143 Essen (DE)
(72) Erfinder: Franzrahe, Harald, 44289 Dortmund (DE)
(74) Vertreter: thyssenkrupp Intellectual Property GmbH

(56) Entgegenhaltungen:
- EP-A1- 3 003 980
- EP-A1- 3 560 907
- EP-A1- 3 562 783
- DE-A1- 102017 203 251
- US-A1- 2020 002 274

## Beschreibung

Die Erfindung betrifft eine Anlage zur Herstellung von Harnstoffgranulat, mit mindestens einem Harnstoffgranulator, mit mindestens einem Staubwäscher, mit mindestens einer Aufkonzentrierungseinrichtung und mit mindestens einer Kondensationseinrichtung, wobei ein Abgasstrom aus dem Harnstoffgranulator dem Staubwäscher zuführbar ist, wobei der Abgasstrom im Staubwäscher gewaschen wird, wobei mindestens ein Ablauf aus dem Staubwäscher der Aufkonzentrierungseinrichtung zuführbar ist, wobei der Ablauf in der Aufkonzentrierungseinrichtung aufkonzentrierbar ist, wobei der beim Aufkonzentrieren entstehende Brüden zumindest teilweise der Kondensationseinrichtung zuführbar ist und wobei der Brüden in der Kondensationseinrichtung zumindest teilweise kondensiert wird.

Daneben betrifft die Erfindung ein Verfahren zur Herstellung von Harnstoffgranulat, wobei ein Abgasstrom aus einem Harnstoffgranulator in einem Staubwäscher gewaschen wird, wobei mindestens ein Ablauf aus dem Staubwäscher aufkonzentriert und dem Harnstoffgranulator zugeführt wird, wobei der beim Aufkonzentrieren entstehende Brüden zumindest teilweise in einer Kondensationseinrichtung kondensiert wird.

Bei der großtechnischen Herstellung von Harnstoff wird beinahe ausschließlich die Hochdrucksynthese von Ammoniak (NH₃) und Kohlenstoffdioxid (CO₂) bei etwa 150 bar und ca. 180 Grad Celsius angewandt. Häufig werden die beiden Einsatzstoffe aus einer benachbarten Ammoniakanlage bezogen.

Für die Herstellung von partikelförmigen, harnstoffhaltigen Zusammensetzungen sind verschiedene Verfahren im Stand der Technik bekannt. In der Vergangenheit wurden Harnstoffpartikel üblicherweise mittels Sprühkristallisation hergestellt, wobei eine im wesentlichen wasserfreie Harnstoffschmelze (Wassergehalt von 0,1 bis 0,3 Gew.-%) vom oberen Teil eines Sprühkristallisationsturmes in einen aufsteigenden Strom von Luft bei Umgebungstemperatur gesprüht werden und sich die Tropfen zu Kristallen (Prills) verfestigen. Die so erhaltenen Prills weisen relativ kleine Durchmesser sowie eine geringe mechanische Festigkeit auf.

Harnstoffpartikel mit größeren Teilchendurchmessern und besseren mechanischen Eigenschaften werden heute zumeist durch Granulierung einer im wesentlichen wasserfreien Harnstoffschmelze oder einer wässrigen Harnstofflösung in einem Fließbett hergestellt. In diesen Granulierungsverfahren wird eine wässrige Harnstofflösung mit einer Harnstoffkonzentration von 70 - 99,9 Gew.-% in Form von sehr fein verteilten Tröpfchen mit einem durchschnittlichen Durchmesser von 20 - 120 Mikrometer in ein Fließbett von Harnstoffpartikeln gegeben, wobei die Temperatur so gewählt ist, dass das Wasser der auf die Harnstoffpartikel aufgesprühten Lösung verdampft und Harnstoff auf den Partikeln abgeschieden wird, so dass ein Granulat mit einer gewünschten Partikelgröße von 2,5 mm und mehr erhalten wird.

Die Abluft aus einem Fließbettgranulator enthält einen mehr oder weniger großen Anteil an Staub. Diese Abluft muss gereinigt werden, bevor sie wieder in die Umwelt gelangen darf. Dies geschieht in den meisten Fällen mit Staubwäschern unterschiedlicher Bauart. Bei den heute üblichen Fließbettgranulierverfahren zur Herstellung von Harnstoffgranulat können zwischen 2 - 5 % des Produktstromes als Staub in die Abluft gelangen. Bei Anlagenkapazitäten zwischen 2,000 und 4,000 Tonnen pro Tag lohnt es sich den abgeschiedenen Staub wieder in den Prozess zu integrieren. In der Regel wird deshalb der Ablauf von den Staubwäschern (ca. 35 - 50 Gew.-% Harnstoff) in einer Eindampfung aufkonzentriert (95 - 99 Gew.-% Harnstoff) bevor die Lösung wieder der Granulierung zugeführt werden kann. Bei reinem Harnstoff, der gut wasserlöslich ist, ist diese Vorgehensweise allgemeiner Stand der Technik.

Allerdings ändern sich die agronomischen Anforderungen an den Harnstoffdünger. Zunehmend werden weitere Nährstoffe benötigt um eine ausreichende Versorgung der Pflanzen zu gewährleiten. Einige dieser Sekundär- und Mikronährstoffe sind allerdings nicht wasserlöslich. Dies ist beispielsweise bei elementarem Schwefel der Fall. Zwar können wasserunlösliche Stoffe meistens ohne große Probleme in den Harnstoff mit eingranuliert werden, diese Stoffe sind aber auch im Staub vorhanden und werden von den Staubwäschern abgeschieden.

Das Konzentrieren von Harnstofflösungen erfolgt in der Regel unter Vakuum in einem Rohrbündelverdampfer. Die aufkonzentrierte Lösung wird wieder dem Granulator zugeführt. Anschließend werden die Brüden in einem Rohrbündelkondensator kondensiert. Das Kondensat kann in den Wäschern zur Sättigung der heißen Abluft aus dem Granulator verwendet werden. Enthält die Wäscher-Lösung zum Beispiel elementare Schwefelpartikel, kann Schwefel, aufgrund des hohen Dampfdrucks, im Eindampfer aus dem festen Zustand in die Gasphase sublimieren. Dieser sublimierte Schwefel setzt sich an jeder kühleren Stelle der Anlage ab und blockiert den Apparat somit mittelfristig.

Vor allem in einem "kalten" Brüdenkondensator ist es relativ schwierig, Schwefel zu entfernen. Üblicherweise werden die Brüden in die Rohrbündel eines Rohrbündelkondensators geleitet.

Damit gelangt der Schwefel in die Kühlrohre und diese blockieren. Eine Reinigung der Rohre ist nur mechanisch möglich. Dies bedingt lange Stand- und Reinigungszeiten.

In EP 3 560 907 A1 wird vorgeschlagen, das Abgas einer Harnstoffgranulierung einer Staubwäsche zu unterziehen, die in der Staubwäsche erhaltene Harnstofflösung aufzukonzentrieren, die wasserhaltige Dampfphase aus der Aufkonzentrierung zu kondensieren und das so erhaltene Kondensat zum schnellen Abkühlen ("Quenchen") des Abgases aus der Harnstoffgranulierung einzusetzen. Auch sind aus dem Stand der Technik unterschiedliche Maßnahmen bekannt, um bei der Harnstoffgranulation bestimmte Arten an Ablagerungen zu vermeiden; so lassen sich gemäß US 2020/002274 A1 Ammoniumsalzablagerungen in einer Lösung vermeiden, in welcher bei der Harnstoffgranulierung anfallender Harnstoffstaub und Ammoniak in einer Staubwäsche aufgenommen werden, indem die Ammoniumsalzkonzentration durch wiederholtes Mischen mit Harnstofflösungen von geringerer Ammoniumsalzkonzentration und Eindampfen der dabei erhaltenen Lösung auf maximal 7 Masse-% eingestellt wird. Das Kondensat der beim Aufkonzentrieren erhaltenen Gasphase enthält noch geringe Mengen an Harnstoff, Ammoniak und Ammoniumsalz und kann zum Auffüllen der Waschlösung für die Staubwäsche eingesetzt werden.

Es ist daher Aufgabe der vorliegenden Erfindung, eine Anlage sowie ein Verfahren zur Herstellung von Harnstoffgranulat anzugeben, bei denen ein Zusetzen von Anlagenteilen durch nicht-wasserlösliche Stoffe möglichst vermieden werden kann.

Diese Aufgabe ist zunächst durch den Patentanspruch 1 dadurch gelöst, dass in der Kondensationseinrichtung mögliche Ablagerungen von nicht-wasserlöslichen Stoffen, insbesondere Schwefel, im laufenden Betrieb aus der Kondensationseinrichtung entfernbar sind, wobei die Aufkonzentrierungseinrichtung strömungstechnisch mit der Kondensationseinrichtung verbunden ist und wobei die strömungstechnische Verbindung beheizbar ist. Nicht-wasserlösliche Stoffe werden durch eine Temperaturerhöhung geschmolzen und lösen sich auf diese Weise von den Bewandungen. Die nicht-wasserlöslichen Stoffe können dabei auch derart bereits aus der Kondensationseinrichtung entfernt werden, dass Ablagerungen gar nicht erst entstehen. Dabei ist vorgesehen, dass die Stoffe, sollten sie an den Bewandungen der Kondensationseinrichtung anhaften, abgewaschen oder mittels Scherkraft durch eine eingebrachte Strömung von der Bewandung gelöst werden. Unter nicht-wasserlöslichen Stoffen ist dabei insbesondere Schwefel zu verstehen, da Schwefel ein immer wichtiger werdender Nährstoff in der Düngemittelindustrie ist.

Je nach gewünschter Endkonzentration (70 - 99.9 Gew.-%) kann die Aufkonzentrierung des Ablaufes in mehreren Stufen erfolgen. Jede Stufe der Aufkonzentrierung wird dann bei einem anderen Druck betrieben. Jede Aufkonzentrierstufe wird mit einer Kondensatorverschaltung versehen. Alternativ kann eine gemeinsame Kondensationstufe eingesetzt werden. Bevorzugt wird die Aufkonzentrierung im Unterdruckbereich betrieben, das heißt der Betriebsdruck ist kleiner als der Umgebungsdruck. Höhere Drücke sind jedoch ebenfalls möglich. Die Anlage kann dann mehrere Aufkonzentrierungseinrichtungen und entsprechend mehrere Abläufe aufweisen.

Erfindungsgemäß ist vorgesehen, dass die Aufkonzentrierungseinrichtung strömungstechnisch mit der Kondensationseinrichtung verbunden ist und dass die strömungstechnische Verbindung beheizbar ist. Bei der strömungstechnischen Verbindung kann es sich um gängige Rohrleitungsverbindungen handeln. Durch die Beheizung der strömungstechnischen Verbindung wird verhindert, dass nicht-wasserlösliche Stoffe bereits in der strömungstechnischen Verbindung so weit abkühlen, dass sie sich in den Rohrleitungen absetzen können.

Eine Ausgestaltung der Erfindung sieht weiterhin vor, dass die strömungstechnische Verbindung mindestens eine Flanschverbindung umfasst. Auch die Flanschverbindung ist beheizbar ausgestaltet, um zu vermeiden, dass sich nicht-wasserlösliche Stoffe in der Flanschverbindung absetzen.

Zur weiteren Verbesserung der Abscheidung, insbesondere von Schwefel, ist bei einer weiteren Ausgestaltung der Erfindung vorgesehen, dass die strömungstechnische Verbindung derart beheizbar ist, dass die Temperatur größer 115 Grad Celsius, vorzugsweise größer 125 Grad Celsius, besonders bevorzugt 135 Grad Celsius ist. Elementarer Schwefel besitzt eine Schmelztemperatur von etwa 115 Grad Celsius. Eine Temperatur über 115 Grad sollte also mindestens erreicht werden. Besonders bevorzugt ist eine Temperatur von etwa 135 Grad Celsius, weil eine Temperaturdifferenz von etwa 20 Grad Celsius das Schmelzen von elementarem Schwefel an den Bewandungen der strömungstechnischen Verbindung gewährleistet.

Zur Verbesserung der Abscheidung von nicht-wasserlöslichen Stoffen ist bei einer weiteren Ausgestaltung der erfindungsgemäßen Anlage vorgesehen, dass die Kondensationseinrichtung mindestens einen ersten Kondensator und einen zweiten Kondensator umfasst, dass die Kondensatoren voneinander unabhängig mit dem Brüden durchströmbar sind und dass im laufenden Betrieb wahlweise der erste Kondensator oder der zweite Kondensator mit Brüden durchströmbar ist. Durch die Verwendung von mindestens zwei Kondensatoren ist es möglich, dass nur ein Kondensator prozesstechnisch in Betrieb ist. Sollten nicht-wasserlösliche Stoffe die Rohre bzw. Bewandungen des ersten Kondensators zusetzen, kann der zweite Kondensator in Betrieb genommen werden. In der Zeit, in der der zweite Kondensator in Betrieb ist, kann der erste Kondensator gereinigt werden. Dabei kann beispielsweise ein Medium zur Wärmeübertragung durch die Kühlrohre des außer Betrieb genommenen Kondensators geleitet werden, das eine ausreichend hohe Temperatur aufweist, damit der festgesetzte nicht-wasserlösliche Stoff schmilzt und von der Bewandung gelöst wird. Bei dem ersten Kondensator und/oder dem zweiten Kondensator kann es sich dabei um einen Rohrbündelkondensator handeln.

Bei einer weiteren bevorzugten Ausgestaltung der Erfindung ist vorgesehen, dass der erste Kondensator oder der zweite Kondensator wahlweise mit Kühlwasser oder mit Dampf betreibbar sind. Hat sich ein nicht-wasserlöslicher Stoff, beispielsweise Schwefel, an der Bewandung eines Kondensators festgesetzt, kann anstatt Kühlwasser Dampf durch die Kühlrohre geleitet werden. Der weiterhin anfallende Brüden kann zum parallelen Kondensator geleitet werden. Wenn Dampf mit einer ausreichend hohen Temperatur durch die Kühlrohre geleitet wird, schmilzt der nicht-wasserlösliche Stoff und löst sich von den Bewandungen des Kondensators. Der Dampf sollte eine Temperatur von mindestens 115 Grad aufweisen, bevorzugt 125 Grad Celsius, besonders bevorzugt etwa 135 Grad Celsius. Elementarer Schwefel hat eine Schmelztemperatur von etwa 115 Grad Celsius. Bei einer Temperatur von 135 Grad Celsius, also einer Temperaturdifferenz von etwa 20 Grad Celsius in Bezug auf die Schmelztemperatur, kann gewährleistet werden, dass Schwefel schmilzt.

Zur weiteren Gestaltung der Anlage ist bei einer weiteren Ausgestaltung vorgesehen, dass der erste Kondensator und/oder der zweite Kondensator als U-Rohr Kondensator ausgestaltet ist/sind.

Bei einer alternativen oder zusätzlichen Ausgestaltung der Erfindung ist vorgesehen, dass die Kondensationseinrichtung mindestens einen Sprühkondensator umfasst. Der Aufbau eines Sprühkondensators ist vergleichsweise einfach. Er besteht aus einem turmartigen Behälter in dem Kühlwasser eingesprüht wird. Durch die hohen Flüssigkeitsmengen wird von vornherein vermieden, dass sich nicht-wasserlösliche Stoffe an den Bewandungen des Sprühkondensators absetzen können.

Für eine effizientere Nutzung der Anlage ist bei einer weiteren bevorzugten Ausgestaltung der Erfindung vorgesehen, dass die Kondensationseinrichtung eine Pumpe umfasst, durch die das Sprühwasser des Sprühkondensators zirkulierbar ist und dass die Kondensationseinrichtung einen Wärmeübertrager umfasst, durch den das Sprühwasser des Sprühkondensators kühlbar ist. So kann das benötigte Sprühwasser bzw. Kühlwasser zirkuliert und wiederverwendet werden. Bei dem Wärmeübertrager kann es sich um einen Plattentauscher handeln. Der aus dem Sprühkondensator austretende Wasserstrom enthält sowohl den beispielsweise abgeschiedenen Schwefel als auch die kondensierten Brüden. Denkbar ist auch, dass eine Durchflussregelung vorgesehen ist, durch die der umlaufende Wasserstrom konstant gehalten werden kann. Das anfallende Kondensatwasser mit beispielsweise Schwefelanteil wird dem Waschsystem zugeführt. Dort dient es als Make-up Flüssigkeit.

Die vorgenannte Aufgabe wird außerdem gelöst von einem Verfahren zur Herstellung von Harnstoffgranulat, wobei ein Abgasstrom aus einem Harnstoffgranulator in einem Staubwäscher gewaschen wird, wobei mindestens ein Ablauf aus dem Staubwäscher aufkonzentriert und dem Harnstoffgranulator zugeführt wird, wobei der beim Aufkonzentrieren entstehende Brüden zumindest teilweise in einer Kondensationseinrichtung kondensiert wird, dadurch gekennzeichnet, dass in der Kondensationseinrichtung mögliche Ablagerungen von nicht-wasserlöslichen Stoffen, insbesondere Schwefel, im laufenden Betrieb aus der Kondensationseinrichtung entfernt werden, wobei die Kondensationseinrichtung mindestens einen Sprühkondensator umfasst und wobei der Brüden von der Aufkonzentrierungseinrichtung zumindest teilweise über eine beheizbare strömungstechnische Verbindung der Kondensationseinrichtung zugeführt wird.

Das Verfahren kann mit einer vorgenannten Anlage durchgeführt werden. Die Ausführungen zur erfindungsgemäßen Anlage gelten entsprechend auch für das erfindungsgemäße Verfahren.

Das erfindungsgemäße Verfahren sieht vor, dass die Kondensationseinrichtung mindestens einen Sprühkondensator umfasst und dass der Brüden von der Aufkonzentrierungseinrichtung zumindest teilweise über eine beheizbare strömungstechnische Verbindung der Kondensationseinrichtung zugeführt wird.

Um insbesondere Schwefel abscheiden zu können, ist bei einer ersten bevorzugten Ausgestaltung des erfindungsgemäßen Verfahrens vorgesehen, dass die beheizbare strömungstechnische Verbindung auf eine Temperatur größer 115 Grad Celsius, bevorzugt größer 125 Grad Celsius, besonders bevorzugt 135 Grad Celsius gebracht wird.

Eine weitere Ausgestaltung des erfindungsgemäßen Verfahrens sieht vor, dass die Kondensationseinrichtung mindestens einen ersten Kondensator und einen zweiten Kondensator umfasst, dass die Kondensatoren voneinander unabhängig mit dem Brüden durchströmt werden können und dass im laufenden Betrieb vom ersten Kondensator auf den zweiten Kondensator und umgekehrt umgeschaltet werden kann.

Bei einer weiteren Ausgestaltung des Verfahrens ist vorgesehen, dass der erste Kondensator oder der zweite Kondensator wahlweise mit Kühlwasser oder mit Dampf betrieben werden kann. Der Dampf hat vorzugsweise einer Temperatur die über der Schmelztemperatur der im Stoffstrom befindlichen nicht-wasserlöslichen Stoffe liegt. Auf diese Weise werden die nicht-wasserlöslichen Stoffe, die sich an der Bewandung des ersten oder zweiten Kondensators abgesetzt haben, aufgeschmolzen und aus dem Kondensator befördert werden. Mit Betreiben des Kondensators mit Kühlwasser oder Dampf ist gemeint, dass entweder Kühlwasser oder Dampf durch die Kühlrohre des Kondensators geleitet wird. Auf diese Weise nimmt entweder das Kühlwasser die Wärme des Stoffstroms auf, der kondensiert wird, oder aber die festgesetzten, nicht-wasserlöslichen Stoffe nehmen die Wärme des Dampfes auf und schmelzen. Es besteht kein Stoffaustausch zwischen dem Medium zur Wärmeübertragung, also dem Dampf oder dem Kühlwasser, und dem Stoffstrom bzw. den nicht-wasserlöslichen Stoffen im Kondensator.

Im Einzelnen gibt es eine Vielzahl von Möglichkeiten, die erfindungsgemäße Anlage und das erfindungsgemäße Verfahren auszugestalten und weiterzubilden. Dazu wird verwiesen sowohl auf die den Patentansprüchen 1 und 9 nachgeordneten Patentansprüche, als auch auf die nachfolgende Beschreibung einer nicht von der Erfindung abgedeckten Anlage sowie eines bevorzugten Ausführungsbeispiels in Verbindung mit der Zeichnung. In der Zeichnung zeigen
- Fig. 1: eine schematische Darstellung eines Teils einer nicht von der Erfindung abgedeckten Harnstoffgranulation mit einer Schwefelrückgewinnung mittels redundanter Wärmeübertrager und
- Fig. 2: eine schematische Darstellung eines Teils einer erfindungsgemäßen Harnstoffgranulation mit einer Schwefelrückgewinnung mittels Sprühkondensation.

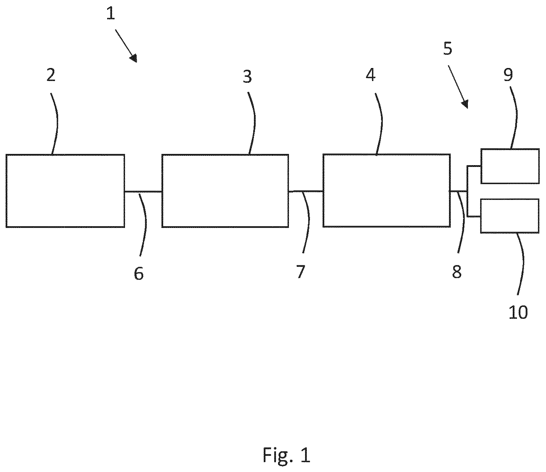

Fig. 1 zeigt eine schematische Darstellung einer nicht von der Erfindung abgedeckten Anlage 1 zur Herstellung von Harnstoffgranulat mittels eines Harnstoffgranulators 2. Die Anlage umfasst unter anderem einen Staubwäscher 3, eine Aufkonzentrierungseinrichtung 4 und eine Kondensationseinrichtung 5. Ein aus dem Harnstoffgranulator 2 austretender Abgasstrom 6 enthält Staub und wird daher dem Staubwäscher 3 zugeführt. Gerade bei der Herstellung von Harnstoffgranulat muss die Abluft, die aus dem Harnstoffgranulator 2 in Form eines Fließbettgranulators austritt, gereinigt werden, bevor sie wieder in die Umwelt gelangen darf. Durch die erhöhten Anforderungen an Düngemittel werden weitere Nährstoffe benötigt, die ohne Probleme in das Produkt eingranuliert werden, sich unter Umständen aber als Feststoff in der Anlage festsetzen können. Einer dieser Nährstoffe ist Schwefel.

Der den Staubwäscher 3 verlassende Ablauf 7 wird der Aufkonzentrierungseinrichtung 4 zugeführt. Mittels Eindampfen wird die Harnstofflösung unter Vakuum in einem hier nicht dargestellten Rohrbündelverdampfer in der Aufkonzentrierungseinrichtung 4 aufkonzentriert. Die aufkonzentrierte Harnstofflösung kann dem Harnstoffgranulator 2 wieder zugeführt werden. Der Brüden 8, der aus der Aufkonzentrierungseinrichtung 4 austritt wird in der Kondensationseinrichtung 5 kondensiert. Da beispielsweise Schwefel einen relativ hohen Dampfdruck aufweist, wird fester Schwefel teilweise in der Aufkonzentrierungseinrichtung 4 in die Gasphase sublimiert und ist ebenfalls im Brüden 8 enthalten Das Kondensat kann wieder zur Sättigung der heißen Abluft aus dem Harnstoffgranulator 2 in dem Staubwäscher 3 verwendet werden.

Bei der in Fig. 1 dargestellten nicht-erfindungsgemäßen Anlage 1 umfasst die Kondensationseinrichtung 5 einen ersten Kondensator 9 und einen zweiten Kondensator 10. Denkbar sind aber auch weitere Kondensatoren. Die Kondensatoren 9, 10 sind als U-Rohr Kondensatoren ausgestaltet. Der erste Kondensator 9 und der zweite Kondensator 10 sind derart miteinander verschaltet, dass der erste Kondensator 9 außer Betrieb genommen werden kann, wobei gleichzeitig der zweite Kondensator 10 in Betrieb genommen wird. Das heißt, dass im laufenden Betrieb vom ersten Kondensator 9 auf den zweiten Kondensator 10 umgeschaltet werden kann. Der heiße Brüden 8 wird beispielsweise im ersten Kondensator 9 kondensiert.

Der Brüden 8 enthält neben Harnstoff auch die weiteren Nährstoffe wie beispielsweise Schwefel. Dieser gasförmige Schwefel kondensiert im ersten Kondensator 9 aus und setzt sich an den kalten Stellen, also an den Bewandungen des ersten Kondensators 9, fest. Wenn der Kondensator 9 soweit zugesetzt ist, dass der Betrieb der Anlage 1 möglicherweise gefährdet ist, kann im laufenden Betrieb auf den zweiten Kondensator 10, an dessen Bewandungen sich kein Schwefel befindet, umgeschaltet werden. Der erste Kondensator 9 und der zweite Kondensator 10 sind so ausgestaltet, dass sie als Medium zur Wärmeübertragung sowohl mit Kühlwasser als auch mit Dampf betreibbar sind. Wenn der erste Kondensator 9 außer Betrieb genommen wird, kann er "regeneriert" werden, indem, anstatt Kühlwasser, Dampf von etwa 135 Grad Celsius durch die Kühlrohre des ersten Kondensators 9 geleitet wird. Der Dampf heizt die Bewandungen des ersten Kondensators 9 soweit auf, dass der Schwefel, der sich an den Bewandungen festgesetzt hat schmilzt und aus dem ersten Kondensator 9 entfernt werden kann. Auf diese Weise ist der erste Kondensator 9 wieder einsatzbereit. Es kann folglich auf den ersten Kondensator 9 umgeschaltet werden, wenn der zweite Kondensator 10 soweit mit nicht-wasserlöslichen Stoffen zugesetzt ist, dass ein Entfernen dieser Stoffe notwendig wird.

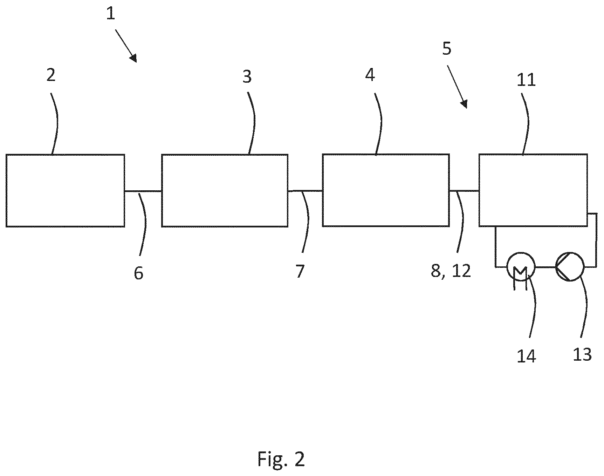

Fig. 2 zeigt einen ähnlichen Aufbau einer Anlage 1 zur Herstellung von Harnstoffgranulat, wie bereits in Fig. 1 beschrieben, jedoch ist die in Fig. 2 dargestellte Anlage erfindungsgemäß. Im Gegensatz zu Fig. 1 umfasst die Kondensationseinrichtung 5 keinen ersten und zweiten Kondensator 9, 10, sondern einen Sprühkondensator 11. Der Sprühkondensator 11 ist ein relativ einfach ausgestalteter Apparat. Er besteht aus einem turmartigen Behälter, in den Kühlwasser eingesprüht wird. Durch die hohen Flüssigkeitsmengen wird in dem Sprühkondensator 11 von vornherein vermieden, dass elementarer Schwefel, der aus der Gasphase kondensiert, an den Bewandungen des Sprühkondensators 11 absetzt.

Der Sprühkondensator 11 bzw. die Kondensationseinrichtung 5 ist mittels einer strömungstechnischen Verbindung 12, in diesem Fall über Rohr- und Flanschverbindungen mit der Aufkonzentrierungseinrichtung 4 verbunden. Die strömungstechnische Verbindung 12 ist beheizt ausgestaltet, damit Schwefel nicht in der strömungstechnischen Verbindung 12 auskondensieren und die Rohr- und Flanschverbindungen zusetzen kann. Im Sprühkondensator 12 wird dies durch das eingesprühte Wasser vermieden. Die Temperatur der strömungstechnischen Verbindung 12 wird auf etwa 135 Grad Celsius eingestellt, damit eine Temperaturdifferenz zur Schmelztemperatur von Schwefel zu etwa 20 Grad Celsius vorherrscht.

Das benötigte Sprühwasser wird mit einer Pumpe 13 zirkuliert und mittels eines Wärmeübertragers 14, in Form eines Plattentauschers, gekühlt. Der aus dem Sprühkondensator 12 austretende Wasserstrom enthält sowohl den abgeschiedenen Schwefel, als auch den kondensierten Brüden 8. Über eine hier nicht dargestellte Durchflussregelung kann erreicht werden, dass der umlaufende Wasserstrom des Sprühkondensators konstant gehalten wird. Das anfallende Kondensatwasser, mit einem Anteil an Schwefel, kann dem Staubwäscher 3 zugeführt werden. Dort dient es als Make-up Flüssigkeit.

### Bezugszeichenliste

- (1): Anlage
- (2): Harnstoffgranulator
- (3): Staubwäscher
- (4): Aufkonzentrierungseinrichtung
- (5): Kondensationseinrichtung
- (6): Abgasstrom
- (7): Ablauf
- (8): Brüden
- (9): Erster Kondensator
- (10): Zweiter Kondensator
- (11): Sprühkondensator
- (12): Strömungstechnische Verbindung
- (13): Pumpe
- (14): Wärmeübertrager

## Patentansprüche

1. Anlage (1) zur Herstellung von Harnstoffgranulat, mit mindestens einem Harnstoffgranulator (2), mit mindestens einem Staubwäscher (3), mit mindestens einer Aufkonzentrierungseinrichtung (4) und mit mindestens einer Kondensationseinrichtung (5), wobei ein Abgasstrom (6) aus dem Harnstoffgranulator (2) dem Staubwäscher (3) zuführbar ist, wobei der Abgasstrom (6) im Staubwäscher (3) gewaschen wird, wobei mindestens ein Ablauf (7) aus dem Staubwäscher (3) der Aufkonzentrierungseinrichtung (4) zuführbar ist, wobei der Ablauf (7) in der Aufkonzentrierungseinrichtung (4) aufkonzentrierbar ist, wobei der beim Aufkonzentrieren entstehende Brüden (8) zumindest teilweise der Kondensationseinrichtung (5) zuführbar ist und wobei der Brüden (8) in der Kondensationseinrichtung (5) zumindest teilweise kondensiert wird,
**dadurch gekennzeichnet,**
**dass** in der Kondensationseinrichtung (5) mögliche Ablagerungen von nicht-wasserlöslichen Stoffen, insbesondere Schwefel, im laufenden Betrieb aus der Kondensationseinrichtung (5) vermieden werden oder zumindest im Betrieb entfernbar sind, wobei die Aufkonzentrierungseinrichtung (4) strömungstechnisch mit der Kondensationseinrichtung (5) verbunden ist und wobei die strömungstechnische Verbindung (12) beheizbar ist.

2. Anlage (1) nach Anspruch 1, **dadurch gekennzeichnet, dass** die strömungstechnische Verbindung (12) mindestens eine Flanschverbindung umfasst.

3. Anlage (1) nach Anspruch 1 oder 2, **dadurch gekennzeichnet, dass** die strömungstechnische Verbindung (12) derart beheizbar ist, dass die Temperatur größer 115 Grad Celsius, vorzugsweise größer 125 Grad Celsius, besonders bevorzugt 135 Grad Celsius ist.

4. Anlage (1) nach einem der Ansprüche 1 bis 3, **dadurch gekennzeichnet, dass** die Kondensationseinrichtung (5) mindestens einen ersten Kondensator (9) und einen zweiten Kondensator (10) umfasst, dass die Kondensatoren (9, 10) voneinander unabhängig mit dem Brüden (8) durchströmbar sind und dass im laufenden Betrieb wahlweise der erste Kondensator (9) oder der zweite Kondensator (10) mit Brüden (8) durchströmbar ist.

5. Anlage (1) nach Anspruch 4, **dadurch gekennzeichnet, dass** der erste Kondensator (9) oder der zweite Kondensator (10) wahlweise mit Kühlwasser oder mit Dampf betreibbar sind.

6. Anlage (1) nach Anspruch 4 oder 5, **dadurch gekennzeichnet, dass** der erste Kondensator (9) und/oder der zweite Kondensator (10) als U-Rohr Kondensator ausgestaltet ist/sind.

7. Anlage (1) nach einem der Ansprüche 1 bis 6, **dadurch gekennzeichnet, dass** die Kondensationseinrichtung (5) mindestens einen Sprühkondensator (11) umfasst.

8. Anlage (1) nach Anspruch 7, **dadurch gekennzeichnet, dass** die Kondensationseinrichtung (5) eine Pumpe (13) umfasst, durch die das Sprühwasser des Sprühkondensators (11) zirkulierbar ist und dass die Kondensationseinrichtung (5) einen Wärmeübertrager (14) umfasst, durch den das Sprühwasser des Sprühkondensators (11) kühlbar ist.

9. Verfahren zur Herstellung von Harnstoffgranulat, wobei ein Abgasstrom (6) aus einem Harnstoffgranulator (2) in einem Staubwäscher (3) gewaschen wird, wobei mindestens ein Ablauf (7) aus dem Staubwäscher (3) aufkonzentriert und dem Harnstoffgranulator (2) zugeführt wird, wobei der beim Aufkonzentrieren entstehende Brüden (8) zumindest teilweise in einer Kondensationseinrichtung (5) kondensiert wird,
**dadurch gekennzeichnet,**
**dass** in der Kondensationseinrichtung (5) mögliche Ablagerungen von nicht-wasserlöslichen Stoffen, insbesondere Schwefel, im laufenden Betrieb aus der Kondensationseinrichtung (5) entfernt werden, wobei die Kondensationseinrichtung (5) mindestens einen Sprühkondensator (11) umfasst und wobei der Brüden (8) von der Aufkonzentrierungseinrichtung (4) zumindest teilweise über eine beheizbare strömungstechnische Verbindung (12) der Kondensationseinrichtung (5) zugeführt wird.

10. Verfahren nach Anspruch 9, **dadurch gekennzeichnet, dass** die beheizbare strömungstechnische Verbindung (12) auf eine Temperatur größer 115 Grad Celsius, bevorzugt größer 125 Grad Celsius, besonders bevorzugt 135 Grad Celsius gebracht wird.

11. Verfahren nach Anspruch 9 oder 10, **dadurch gekennzeichnet, dass** die Kondensationseinrichtung (5) mindestens einen ersten Kondensator (9) und einen zweiten Kondensator (10) umfasst, dass die Kondensatoren (9, 10) voneinander unabhängig mit dem Brüden (8) durchströmt werden können und dass im laufenden Betrieb vom ersten Kondensator (9) auf den zweiten Kondensator (10) und umgekehrt umgeschaltet werden kann.

12. Verfahren nach Anspruch 11, **dadurch gekennzeichnet, dass** der erste Kondensator (9) oder der zweite Kondensator (10) wahlweise mit Kühlwasser oder mit Dampf betrieben werden kann.

## Claims

1. Installation (1) for the production of granular urea, having at least one urea granulator (2), at least one dust scrubber (3), at least one concentrating device (4), and at least one condensation device (5), wherein an exhaust gas flow (6) from the urea granulator (2) can be fed to the dust scrubber (3), wherein the exhaust gas flow (6) is washed in the dust scrubber (3), wherein at least one outflow (7) from the dust scrubber (3) can be fed to the concentrating device (4), wherein the outflow (7) can be concentrated in the concentrating device (4), wherein the vapors (8) created during concentration can be fed, at least in part, to the condensation device (5), and wherein the vapors (8) are at least partially condensed in the condensation device (5),
**characterized in that**
possible deposits of non-water-soluble substances, in particular sulfur, in the condensation device (5) are avoided during ongoing operation from the condensation device (5) or are at least removable during operation, wherein the concentrating device (4) is fluidically connected to the condensation device (5), and wherein the fluidic connection (12) can be heated.

2. Installation (1) according to claim 1, **characterized in that** the fluidic connection (12) comprises at least one flange connection.

3. Installation (1) according to claim 1 or 2, **characterized in that** the fluidic connection (12) can be heated in such a manner that the temperature is greater than 115 degrees Celsius, preferably greater than 125 degrees Celsius, particularly preferably 135 degrees Celsius.

4. Installation (1) according to one of claims 1 to 3, **characterized in that** the condensation device (5) comprises at least a first condenser (9) and a second condenser (10), that vapors (8) can flow through the condensers (9, 10) independently of one another, and that during ongoing operation, vapors (8) can selectively flow either through the first condenser (9) or through the second condenser (10).

5. Installation (1) according to claim 4, **characterized in that** the first condenser (9) or the second condenser (10) can be selectively operated either with cooling water or with steam.

6. Installation (1) according to claim 4 or 5, **characterized in that** the first condenser (9) and/or the second condenser (10) is/are designed as a U-tube condenser.

7. Installation (1) according to one of claims 1 to 6, **characterized in that** the condensation device (5) comprises at least one spray condenser (11).

8. Installation (1) according to claim 7, **characterized in that** the condensation device (5) comprises a pump (13) through which the spray water of the spray condenser (11) can be circulated, and that the condensation device (5) comprises a heat exchanger (14) through which the spray water of the spray condenser (11) can be cooled.

9. Method for the production of granular urea, wherein an exhaust gas flow (6) from a urea granulator (2) is washed in a dust scrubber (3), wherein at least one outflow (7) from the dust scrubber (3) is concentrated and fed to the urea granulator (2), wherein the vapors (8) produced during concentration are condensed at least in part in a condensation device (5), **characterized in that**
possible deposits of non-water-soluble substances, in particular sulfur, in the condensation device (5) are removed from the condensation device during ongoing operation, wherein the condensation device (5) comprises at least one spray condenser (11) and wherein the vapors (8) from the concentrating device (4) are fed at least in part via a heatable fluidic connection (12) to the condensation device (5).

10. Method according to claim 9, **characterized in that** the heatable fluidic connection (12) is brought to a temperature greater than 115 degrees Celsius, preferably greater than 125 degrees Celsius, particularly preferably 135 degrees Celsius.

11. Method according to claim 9 or 10, **characterized in that** the condensation device (5) comprises at least a first condenser (9) and a second condenser (10), that vapors (8) can flow through the condensers (9, 10) independently of one another, and that it is possible to switch from the first condenser (9) to the second condenser (10), and vice versa during ongoing operation.

12. Method according to claim 11, **characterized in that** the first condenser (9) or the second condenser (10) can be selectively operated either with cooling water or with steam.

## Revendications

1. Installation (1) pour la production de granulés d'urée, comprenant au moins un granulateur d'urée (2), comprenant au moins un laveur de poussières (3), comprenant au moins un dispositif de concentration (4) et comprenant au moins un dispositif de condensation (5), dans laquelle un flux de gaz d'échappement (6) provenant du granulateur d'urée (2) peut être amené au laveur de poussières (3), dans laquelle le flux de gaz d'échappement (6) est lavé dans le laveur de poussières (3), dans laquelle au moins un écoulement (7) provenant du laveur de poussières (3) peut être amené au dispositif de concentration (4), dans laquelle l'écoulement (7) peut être concentré dans le dispositif de concentration (4), dans laquelle les vapeurs (8) générées lors de la concentration peuvent être amenées au moins en partie au dispositif de condensation (5) et dans laquelle les vapeurs (8) sont condensées au moins en partie dans le dispositif de condensation (5),
**caractérisée en ce que**
des dépôts possibles de substances non solubles dans l'eau, en particulier du soufre, dans le dispositif de condensation (5) sont évités pendant le fonctionnement en cours depuis le dispositif de condensation (5) ou sont au moins éliminables pendant le fonctionnement, le dispositif de concentration (4) étant relié de manière fluidique au dispositif de condensation (5) et la liaison fluidique (12) pouvant être chauffée.

2. Installation (1) selon la revendication 1, **caractérisée en ce que** la liaison fluidique (12) comprend au moins une liaison à bride.

3. Installation (1) selon la revendication 1 ou 2, **caractérisée en ce que** la liaison fluidique (12) peut être chauffée de telle sorte que la température est supérieure à 115 degrés Celsius, de préférence supérieure à 125 degrés Celsius, particulièrement de préférence 135 degrés Celsius.

4. Installation (1) selon l'une des revendications 1 à 3, **caractérisée en ce que** le dispositif de condensation (5) comprend au moins un premier condenseur (9) et un second condenseur (10), que les condenseurs (9, 10) peuvent être traversés indépendamment l'un de l'autre par les vapeurs (8) et que, pendant le fonctionnement en cours, soit le premier condenseur (9) soit le second condenseur (10) peut être traversé par les vapeurs (8).

5. Installation (1) selon la revendication 4, **caractérisée en ce que** le premier condenseur (9) ou le second condenseur (10) peut être exploité au choix avec de l'eau de refroidissement ou avec de la vapeur.

6. Installation (1) selon la revendication 4 ou 5, **caractérisée en ce que** le premier condenseur (9) et/ou le second condenseur (10) est/sont conçu(s) comme un condenseur à tubes en U.

7. Installation (1) selon l'une des revendications 1 à 6, **caractérisée en ce que** le dispositif de condensation (5) comprend au moins un condenseur à pulvérisation (11).

8. Installation (1) selon la revendication 7, **caractérisée en ce que** le dispositif de condensation (5) comprend une pompe (13) par laquelle l'eau de pulvérisation du condenseur à pulvérisation (11) peut être mise en circulation et **en ce que** le dispositif de condensation (5) comprend un échangeur de chaleur (14) par lequel l'eau de pulvérisation du condenseur à pulvérisation (11) peut être refroidie.

9. Procédé pour la production de granulés d'urée, dans lequel un flux de gaz d'échappement (6) provenant d'un granulateur d'urée (2) est lavé dans un laveur de poussières (3), dans lequel au moins un écoulement (7) provenant du laveur de poussières (3) est concentré et amené au granulateur d'urée (2), dans lequel les vapeurs (8) produites lors de la concentration sont condensées au moins en partie dans un dispositif de condensation (5),
**caractérisé en ce que,**
des dépôts possibles de substances non solubles dans l'eau, en particulier du soufre, dans le dispositif de condensation (5) sont éliminés pendant le fonctionnement en cours depuis le dispositif de condensation (5), le dispositif de condensation (5) comprenant au moins un condenseur à pulvérisation (11) et les vapeurs (8) étant amenées depuis le dispositif de concentration (4) au moins en partie via une liaison fluidique chauffable (12) au dispositif de condensation (5).

10. Procédé selon la revendication 9, **caractérisé en ce que** la liaison fluidique chauffable (12) est amenée à une température supérieure à 115 degrés Celsius, de préférence supérieure à 125 degrés Celsius, particulièrement de préférence 135 degrés Celsius.

11. Procédé selon la revendication 9 ou 10, **caractérisé en ce que** le dispositif de condensation (5) comprend au moins un premier condenseur (9) et un second condenseur (10), que les condenseurs (9, 10) peuvent être traversés indépendamment l'un de l'autre par les vapeurs (8) et que, pendant le fonctionnement en cours, il est possible de commuter du premier condenseur (9) au second condenseur (10) et inversement.

12. Procédé selon la revendication 11, **caractérisé en ce que** le premier condenseur (9) ou le second condenseur (10) peut être exploité au choix avec de l'eau de refroidissement ou avec de la vapeur.
